# EUROPEAN PATENT SPECIFICATION

(11) **EP 4 313 039 B1**
(45) Date of publication and mention of the grant of the patent: **03.09.2025**
(21) Application number: 22717008.1
(22) Date of filing: 30.03.2022
(51) Int. Cl.: A61K 31/435, A61P 25/02, A61P 1/16, A61P 3/04, A61P 3/06, A61P 3/08, A61P 3/10, A61P 9/10, A61P 13/00, A61P 27/00

(54) **BENZOMORPHAN DERIVATE FOR USE IN THERAPY**
BENZOMORPHAN-DERIVAT ZUR VERWENDUNG IN DER THERAPIE
DÉRIVÉ DE BENZOMORPHANE À UTILISER EN THÉRAPIE

(30) Priority: 30.03.2021 IT 202100007730
(43) Date of publication of application: 07.02.2024
(73) Proprietor: Molecular Research Pharmact S.r.l., 73024 Maglie (LE) (IT)
(72) Inventor: GUCCIONE, Salvatore, 73024 Maglie (LE) (IT); RONSISVALLE, Simone, 73024 Maglie (LE) (IT); BASILE, Livia, 73024 Maglie (LE) (IT); PAPPALARDO, Matteo, 73024 Maglie (LE) (IT); MILARDI, Danilo, 73024 Maglie (LE) (IT); SPADARO, Angelo, 73024 Maglie (LE) (IT); NG, Chun Laam, 73024 Maglie (LE) (IT)
(74) Representative: Fiammenghi, Eva
(86) International application number: PCT/IB2022/052928
(87) International publication number: WO 2022/208375

(56) References cited:
- US-A1- 2011 230 452
- RONSISVALLE SIMONE ET AL: "Molecular modeling and biological studies show that some mu-opioid receptor agonists might elicit analgesia acting as MMP-9 inhibitors", vol. 11, no. 11, 31 May 2019 (2019-05-31), pages 1245 - 1258, XP009531535, ISSN: 1756-8927, Retrieved from the Internet <URL:https://www.future-science.com/doi/10.4155/fmc-2018-0535> [retrieved on 20220613], DOI: 10.4155/FMC-2018-0535

## Description

### Field of the invention

The present description relates to the pharmaceutical sector. More in detail, the present description relates to a specific use of a benzomorphan derivative in the pharmaceutical field. Even more in detail, the present invention relates to the use of a compound, known as an analgesic, for use in the treatment of a disease or disorder capable of being treated by an agonist of AdipoR such as diabetic neuropathy, therefore in addition to its analgesic properties, also used as an activator of adiponectin receptors e.g. in subjects suffering from diabetic neuropathy.

### Background

Diabetic neuropathy is a common, painful and severe complication of diabetes with a multifactorial etiology which comprises oxidative stress, mitochondrial dysfunction, neuronal damage, autoimmunity, inflammation, and ultimately apoptosis, which can affect up to 50% of people with diabetes. Diabetic neuropathy affects peripheral sensory neurons and occurs in a large percentage of adult diabetic patients.

Diabetic neuropathy is a type of nerve damage that can occur in diabetes. High blood sugar (glucose) can damage, and ultimately kill, nerves throughout the body, most often in the legs and feet.

Depending on the nerves affected, the symptoms of diabetic neuropathy can range from pain and numbness in the legs and feet to problems with the digestive system, urinary tract, blood vessels, and heart.

Some people show mild symptoms but for others, diabetic neuropathy can be quite painful and disabling.

Treatment of diabetic neuropathy is still underdeveloped and currently focuses only on symptomatic and variable pain therapies that show varying levels of effectiveness.

For the purposes of understanding the invention which is the subject of this document, it is of interest to point out that adipokines (from the Greek adipo-, fat; cyto-, cell; and -kinos, movement) are cytokines, that is, signaling protein molecules between cell and cell, produced and secreted by adipose tissue.

Adiponectin is the most abundant plasma adipokine that regulates metabolism through glycemic control and fatty acid oxidation. Adiponectin and its receptors (AdipoR) were initially characterized for their role in lipid and glucose metabolism. More recently, adiponectin has been shown to exhibit anti-inflammatory effects and participate in brain homeostasis and neuroprotection.

The compound AdipoRon (IUPAC name: 2-(4-benzoylphenoxy)-*N*-(1-benzylpiperidin-4-yl)acetamide) is a known non-selective agonist of Adipo-R (adiponectin receptors) and has been reported as being useful for treating obesity, type II diabetes (Okada-Iwabu et al.. Nature 503, 493-499. (2013)) diabetic nephropathy (Kim et al., Journal of the American Society of Nephrology 29(4), 1108-1127. (2018)), diabetic retinopathy (Fu et al., Biochimica et biophysica acta, 1862(8), 1392-1400. (2016)), and atherosclerosis (Yamauchi et al., The Journal of Biological chemistry 278, 4, 2461-2468 (2003)) and ameliorating insulin resistance and dyslipidaemia (Iwabu et al., Frontiers in cardiovascular medicine 6, 116 (2019))

The present invention results from the pharmacological evaluation of a known compound *4-(8-hydroxy-6,11-dimethyl-1,4,5,6-tetrahydro-2,6-methane-3-benzazocin-3(2H)-yl)-N,N-dimethyl-2,2-diphenylbutanamide,* a benzomorphan derivative endowed with analgesic activity, which has revealed surprising new pharmacological activities.

Multi-target action can present opportunities in complex pathologies.

US2011/230452 discloses MMP-2 and/or MMP-9 inhibiting compounds for the treatment of inter alia diabetic neuropathy.

### Description of the invention

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

The present description relates to further uses in the pharmaceutical field of a known compound in the treatment of certain specific pathologies.

More in detail, the present invention consists in a use of a benzomorphan derivative, previously indicated as an analgesic.

Even more in detail, the present invention relates to the use of the compound *4-(8-hydroxy-6,11-dimethyl-1,4,5,6-tetrahydro-2,6-methane-3-benzazocin-3(2H)-yl)-N,N-dimethyl-2,2-diphenylbutanamide,* in the treatment of a disease or disorder, as defined in the claims, capable of being treated by an agonist of adiponectin receptors (AdipoR) having proved to be an effective activator of Adipo-R.

Specifically, the intuition behind the present invention lies in the evaluation of a structural similarity perceived by the Inventors between the compound of formula (1) and AdipoRon^{®}, a known agonist of said receptors, supported by analysis via docking of the possible binding to said receptors (Adipo-R), and in the recent demonstration of the involvement of adiponectin in the metabolism of lipids and glucose as well as in anti-inflammatory processes, and its important role as an agent that contributes to cerebral homeostasis and neuroprotection.

As mentioned above, considering the perceived structural similarities with AdipoRon^{®}, a well-known non-selective agonist of Adipo-R (adiponectin receptors) (AdipoR1> AdipoR2), it was also thought in the light of *in silico* evidence (docking) in support of the intuition, to extend the pharmacological evaluation of the compound 4-(8-hydroxy-6,11-dimethyl-1,4,5,6-tetrahydro-2,6-methane-3-benzazocin-3(2H)-yl)-N,N-dimethyl-2,2 [compound of formula (1)], a benzomorphan derivative endowed with analgesic activity, to said adiponectin receptors.

As will be more clearly described below, the compound of formula (1) has been shown to be a potent Adipo-R agonist by increasing the *in vitro* levels of phosphorylated AMPK on HT-22 cells and representing the first case of a multi-target compound with analgesic and Adipo-R agonist binding properties. The compound of formula (1) has also surprisingly been found to protect neurons against high glucose-induced apoptosis. This dual activity of the compound of formula (1) that makes it particularly suitable for the treatment of diabetic neuropathy.

It is also of interest to point out that in the course of the present description, the name of the compound whose particular use in the medical field is the subject of the present description will be indicated with *"SS-SRGR- (*+*7)*" only for simplification, however it is understood that the compound a referred to is 4-(8-hydroxy-6,11-dimethyl-1,4,5,6-tetrahydro-2,6-methane-3-benzazocin-3(2H)-yl)-N,N-dimethyl-2,2-diphenylbutanamide [compound of formula (1)]. The compound 4-(8-hydroxy-6,11-dimethyl-1,4,5,6-tetrahydro-2,6-methane-3-benzazocin-3(2H)-yl)-N,N-dimethyl-2,2-diphenylbutanamide has previously been referred to in the literature as (-)-MML1017 (S. Ronsisvalle et al., Future. Med. Chem. 11(11) 1245-1258 (2019*)).*

### Description of the figures

The invention which is the subject of the present document will be described in detail below also with reference to the accompanying figures, in which:

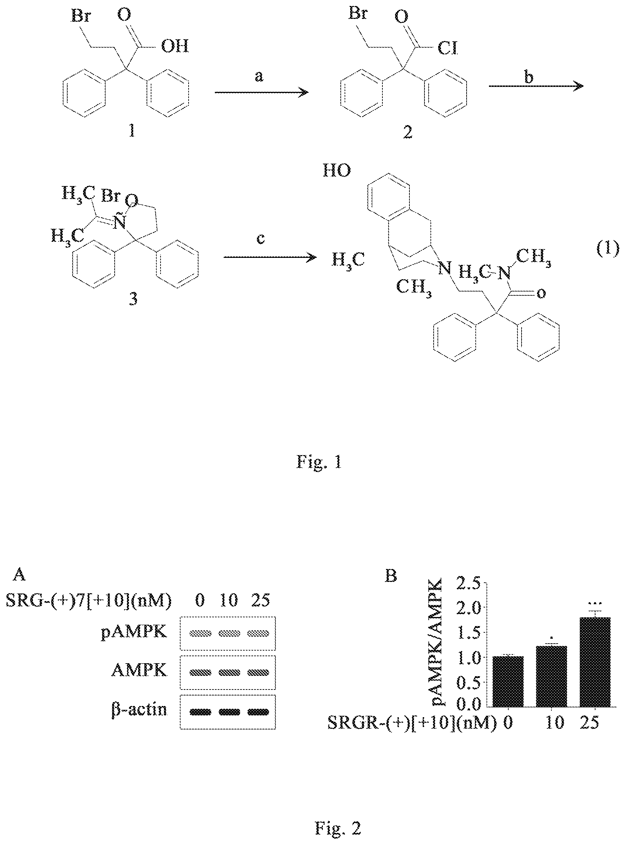

FIGURE 1 shows the synthesis scheme of the compound 4-(8-hydroxy-6,11-dimethyl-1,4,5,6-tetrahydro-2,6-methane-3-benzazocin-3(2H)-yl)-N,N-dimethyl-2,2-diphenylbutanamide [compound of formula (1)]. Reagents and conditions: (A) SO₂Cl₂, Dimethylformamide (DMF) in traces, Chloroform (CHCl₃); (B) Dimethylamine (CH₃)₂NH (2M solution in Tetrahydrofuran (THF), Potassium Carbonate K₂CO₃, Toluene, (C) cis-(-)-(1R,5R,9R)-N-normetazocine.
FIGURE 2 shows the evaluation of SS-SRGR - (+ 7) to induce the adiponectin signal in the neuronal cell line. A. Concentration-dependent activation of AMPK, the downstream intracellular mediator of adiponectin receptors. B. Densitometric analysis of the pAMPK/AMPK ratio under induction by SS-SRGR - (+ 7).

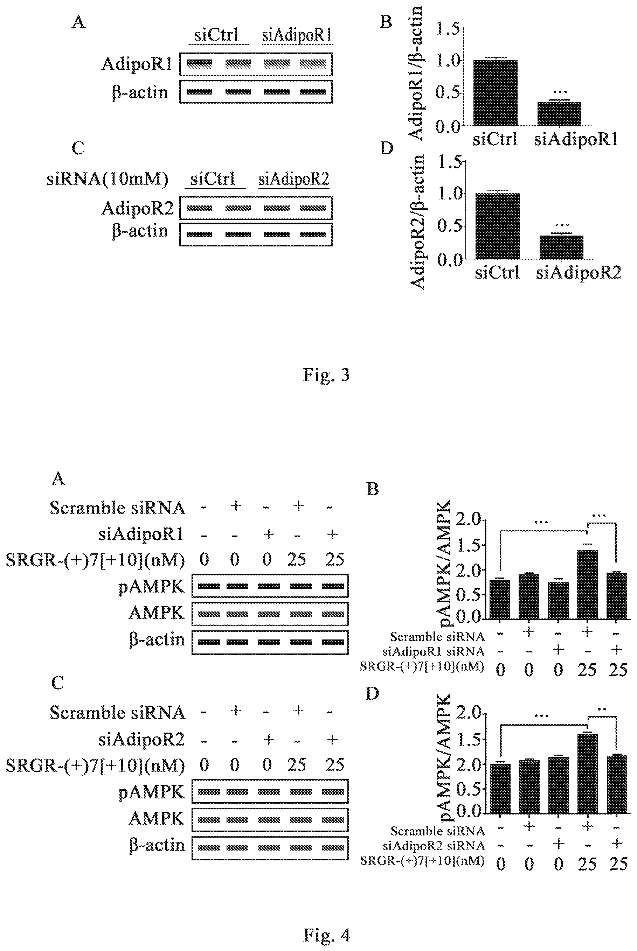

FIGURE 3 shows the suppression of adiponectin receptors by siRNA transfection. A. Western blot image representative of AdipoR1 from HT22 cells transfected with siCtrl RNA or siAdipoR1. B. Densitometric analysis of the AdipoR1 expression in which β-actin was used to standardize the amount of protein in each group. C. Western blot image representative of AdipoR2 of HT22 cells transfected with siCtrl RNA or siAdipoR1. D. Densitometric analysis of the AdipoR2 expression in which β-actin was used to standardize the amount of protein in each group.
FIGURE 4 shows the suppression of AMPK activation, under AdipoR1 and AdipoR2 knock-down conditions, by SS-SRGR - (+ 7) in HT22 neuronal cells. A. Western blot image representative of pAMPK and AMPK in siCtrl transfected HT22 cells and siAdipoR1 transfected HT22 cells with or without SS-SRGR - (+ 7) incubation. B. Densitometric analysis of the pAMPK level where AMPK was used to standardize the amount of protein in each group. C. Western blot image representative of pAMPK and AMPK in siCtrl transfected HT22 cells and siAdipoR2 transfected HT22 cells with or without SS-SRGR - (+ 7) incubation. D. Densitometric analysis of the pAMPK level where AMPK was used to standardize the amount of protein in each group.

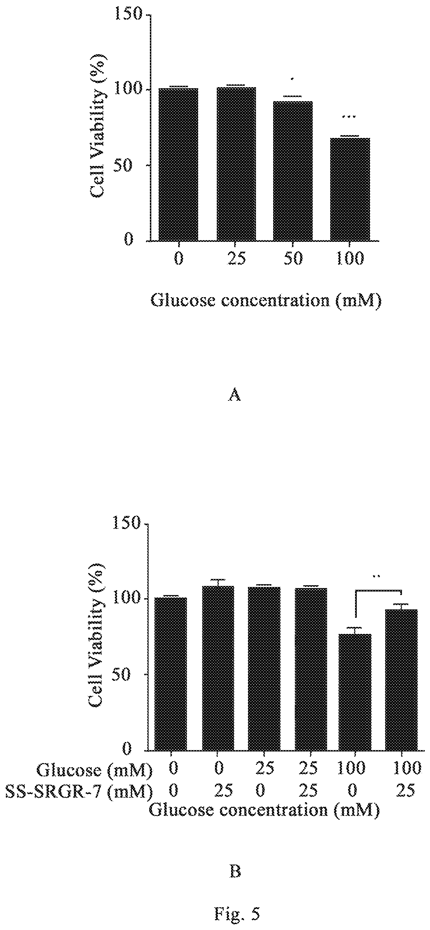

FIGURE 5 shows the neuronal protective capacity of benzomorphan derivatives in conditions of hyperglycemia. 5A: Viability of NSC-34 cells in the presence of 100 mM glucose; 5B: Viability of NSC-34 cells in the presence of SS-SRGR - (+ 7) . SS-SRGR - (+ 7) protects NSC-34 (hybrid cell line) from hyperglycemia-induced apoptosis.

### Detailed description of the invention

On the basis of the results described herein, pharmaceutical compositions and their formulations have been devised and prepared comprising the compound of 4-(8-hydroxy-6,11-dimethyl-1,4,5,6-tetrahydro-2,6-methane-3-benzazocin-3(2H)-yl)-N,N-dimethyl-2,2-diphenylbutanamide and pharmaceutically acceptable excipients. One or more excipients appropriate for the route of administration may be provided.

As repeatedly mentioned, said compound, as well as the pharmaceutical preparations comprising it, are intended for use in the treatment of diabetic neuropathy especially in mammals and in particular in humans.

Said composition can be formulated as: solutions, suspensions, powders, granules, tablets, pills, capsules, syrups, suppositories, sprays, aerosols or controlled-release systems, cream, ointment or gel, lubricants, pastes, syrups, ampoules, phials, drops, eye drops, aerosols acceptable for medical use.

The compositions can be administered by various routes, in particular by the oral, transdermal, subcutaneous, intravenous, intramuscular, rectal and intranasal routes.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. Immediate release or extended release can be achieved by the use of suitable pharmaceutical compositions comprising Compound (1), or, particularly in the case of extended release, by the use of devices such as subcutaneous implants or osmotic pumps.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate, calcium sulfate, sorbitol, glucose and/or lactose and/or other excipients, binders, extenders, disintegrants, diluents and lubricants such as those known in the art. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Disintegrators include without limitation starch, methylcellulose, agar, bentonite, xanthan gum and the like. The Compound of the invention can also be delivered through the oral cavity by sublingual and/or buccal administration. Molded tablets, compressed tablets or freeze-dried tablets are exemplary forms which may be used. Exemplary compositions include those formulating the present compound with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use. Lubricants used in these dosage forms include sodium oleate, sodium stearate, magnesium stearate, sodium benzoate, sodium acetate, sodium chloride and the like. For oral administration in liquid form, the oral drug components can be combined with any oral, non-toxic, pharmaceutically acceptable inert carrier such as ethanol, glycerol, water, and the like. Compositions for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Exemplary compositions for intranasal administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with the usual carriers such as cocoa butter, synthetic glyceride esters or polyethylene glycol. Such carriers are typically solid at ordinary temperatures, but liquefy and/or dissolve in the rectal cavity to release the drug.

One aspect of the invention includes a compound of formula (1) or a pharmaceutical composition comprising a compound of formula (1) for use in the treatment of a disease or disorder capable of being treated by an agonist of AdipoR. A further aspect of the invention is a method of treatment of a disease or disorder capable of being treated by an agonist of AdipoR which comprises administering to a patient in need thereof an effective amount of the compound of formula (I) or a pharmaceutical composition comprising the compound of formula (1). There is also provided use of a compound of formula (I) or a pharmaceutical composition comprising the compound of formula (1) in the manufacture of a medicament for the treatment of a disease or disorder capable of being treated by an agonist of AdipoR. As used herein, "AdipoR" means adiponectin receptors, for example, AdipoR1 and/or AdipoR2. In one aspect of the invention AdipoR is AdipoR1 i.e. diseases or disorders are capable of being treated by an agonist of AdipoR1. In one aspect of the invention AdipoR is AdipoR2 i.e. diseases or disorders are capable of being treated by an agonist of AdipoR2. In one aspect of the invention AdipoR is AdipoR1 and AdipoR2 i.e. diseases or disorders are capable of being treated by an agonist of AdipoR1 and AdipoR2.

One aspect of the invention includes a compound of formula (1) or a pharmaceutical composition comprising a compound of formula (1) for use in the treatment of a metabolic disease or disorder. In a further aspect the disease or disorder is selected from obesity, atherosclerosis, dyslipidaemia (including hypercholesterolaemia or hypertriglyceridaemia) and non-alcoholic fatty liver disease. In a further aspect the disease or disorder comprises hyperglycaemia. In a further aspect the disease or disorder is selected from diabetes e.g. Type I or Type II diabetes, especially Type II diabetes, diabetic nephropathy and diabetic retinopathy. In a further specific aspect the disease or disorder is diabetic neuropathy.

There is also provided the compound of formula (1) or a pharmaceutical composition comprising a compound of formula (1) for use in treatment of a disease or disorder capable of being treated by an agonist of AdipoR in combination with a second or further medicine for the treatment of a disease or disorder capable of being treated by an agonist of AdipoR. There is also provided a method of treatment of a disease or disorder capable of being treated by an agonist of AdipoR which comprises administering to a patient in need thereof an effective amount of the compound of formula (I) in combination with a second or further medicine for the treatment of a disease or disorder capable of being treated by an agonist of AdipoR. The compound of formula (I) and the second or further medicine may be administered simultaneously (e.g. are co-formulated) or separately. A suitable dose of such a suitable second or further medicine will be known or can be easily determined by the skilled person.

When the disease or disorder capable of being treated by an agonist of AdipoR is obesity, the second or further medicine may be selected from amphetamine, benzphetmaine, bupropion, delamanid, diethylpropion, fluoxetine, lecithin, lorcaserin, naltrexone, orlistat, phendimetrazine, phentermine, pipradrol, and sibutramine.

When the disease or disorder capable of being treated by an agonist of AdipoR is atherosclerosis, the second or further medicine may be selected from acetylsalicylic acid, amlodipine, atorvastatin, bempedoic acid, cholestyramine, fluvastatin, gemfibrozil, inclisiran, lovastatin, picotamide, pravastatin, pyridoxine, rivaroxaban, rosuvastatin, and ticagrelor.

When the disease or disorder capable of being treated by an agonist of AdipoR is non-alcoholic fatty liver disease, the second or further medicine may be selected from aramchol, cenicriviroc, cotadutide, elafibranor, emricasan, GR-MD-02, MSDC-0602K, NGM282, obeticholic acid, pioglitazone, resmetirom, saroglitazar, selonsertib, semaglutide, silibinin, simtuzumab, tirzepatide, and tropifexor.

When the disease or disorder capable of being treated by an agonist of AdipoR is dyslipidaemia, the second or further medicine may be selected from statins such as atorvastatin, rosuvastatin, simvastatin, fluvastatin and pravastatin, fenofibrate, exetimibe, evolocumab, cholestyramine, cholestipol (e.g. as HCl) and alirocumab.

When the disease or disorder capable of being treated by an agonist of AdipoR is diabetes, the second or further medicine may be selected from acarbose, albiglutide, alogliptin, atorvastatin becaplermin, bromocriptine, canagliflozin, captopril, chlorpropamide, colesevelam, dapagliflozin, dasiglucagon, dulaglutide, empagliflozin, ertuglitlozin, exenatide, gemigliptin, gliclazide, glimepiride, glipizide, gliquidone, glucagon, glyburide, icosapent ethyl, insulin aspart, insulin degludec, insulin detemir, insulin glargine, insulin glulisine, insulin human, insulin lispro, linagliptin, lipoic acid, liraglutide, lixisenatide, lisinopril, louseoglitlozin, metformin, miglitol, mitiglinide, nateglinide, pioglitazone, pramlintide, repaglinide, rosiglitazone, saxagliptin, semaglutide, simvastatin, sitagliptin, sotagliflozin, teneligliptin, tolazamide, tolbutamide, trelagliptin, vildagliptin, and voglibose.

When the disease or disorder capable of being treated by an agonist of AdipoR is diabetic nephropathy, the second or further medicine may be selected from benazepril, canagliflozin, candesartan cilexetil, captopril, enalapril, enalaprilat, eprosartan, irbesartan, lisinopril, losartan, olmesartan, perindopril, quinapril, ramipril, telmisartan, and valsartan.

When the disease or disorder capable of being treated by an agonist of AdipoR is diabetic retinopathy, the second or further medicine may be selected from aflibercept, and ranibizumab. When the disease or disorder capable of being treated by an agonist of AdipoR is diabetic neuropathy, the second or further medicine may be selected from amitriptyline, botulinum toxin A, citalopram, desipramine, duloxetine, lipoic acid, pioglitazone, pregabalin, and tapentadol.

The compound of formula (I) and/or any of the second or further medicines mentioned above may be administered in the form of a salt or a solvate or solvate of a salt thereof. Such salts and/or solvates are suitable physiologically acceptable.

The compound of formula (1) may be administered 1-4 times daily e.g. 1 or 2 times daily. A suitable dose or effective amount of the compound of formula (I) for an average adult may be determined by the skilled person and is, for example, 10-1000 µg per day.

### Examples

The synthesis of 4-(8-hydroxy-6,11-dimethyl-1,4,5,6-tetrahydro-2,6-methane-3-benzazocin-3(2H)-yl)-N,N-dimethyl-2,2-diphenylbutanamide was conducted as reported in the literature (S. Ronsisvalle et al., Future. Med. Chem. 11(11) 1245-1258 (2019*):* 7 *[(*+*) - MML1017]*). Specifically, with reference to Figure 1, the intermediates 2 and 3 were synthesized as previously described by Stokbroekx et al. (J. Med. Chem. 16(7), 782-786 (1973).

A mixture of cis - (+) - (1S,5S,9S)-N-normetazocine (150.00 mg, 0.69 mmol), dimethyl-(tetrahydro-3,3-diphenyl-2-furylidene)-ammonium bromide 3 (263.00 mg, 0.76 mmol), anhydrous K₂CO₃ (290 mg, 2.10 mmol) in CH₃CN (40 ml) was heated under reflux for 16 hours in an argon atmosphere in the dark, subsequently cooled to room temperature and the solvent removed at reduced pressure. The solid residue obtained was then added with 50 ml of H₂O and extracted with dichloromethane (3 × 50 ml). The combined organic extracts were washed with *brine* (a solution with a high concentration of Sodium Chloride in water) and dried over anhydrous Na₂SO₄. Evaporation under reduced pressure gave the compound, which was purified by flash chromatography on silica gel, using a CH₂Cl₂ (dichloromethane)/methanol mixture as eluent. The suitable fractions were combined and the solvent was removed under reduced pressure to give the compound as a colorless crystalline solid (140 mg, 0.29 mmol, 42%). 1H NMR (CDCl3): 7.24-7.45 (m, 10H), 9.52 (s, 1H), 6.96-6.92 (m, 1H), 6.77-6.74 (m, 1H), 6.69-6.64 (m, 1H), 2.95 (s, 3H), 2.90-2.82 (m, 3H), 2.75 (m, 2H), 2.64 (m, 2H), 2.50-2.24 (m, 2H), 2.35 (s, 3H), 1.97 -1.71 (m, 2H), 1.42-1.39 (m, 1H), 1.36 (s, 3H), 0.87 (d, J = 5 Hz, 3H). (+) -

### Experiment 1: Concentration-dependent activation of AMPK by SS-SRGR - (+ 7) 1.

### 1. Materials and methods

### 1.1 Antibody

A rabbit anti-phospho-AMPK polyclonal antibody (pAMPK) (Cell Signaling Technology, Ltd.), a rabbit anti-AMPK polyclonal antibody (Cell Signaling Technology, Ltd.) and an HRP conjugated mouse monoclonal anti-β-actin antibody (Cell Signaling Technology, Ltd.) were used as primary antibodies after being suitably diluted. An HRP-labeled rabbit IgG goat antibody (Dako, Glostrup, Denmark) was used as a secondary antibody.

### 1.2 Cell culture

Under conditions of 5% CO₂ at 37 °C, mouse neuronal cells (HT22) were maintained and expanded in a Dulbecco-modified high-glucose Eagle medium (DMEM, GIBCO, Invitrogen, Life Technologies Hong Kong Ltd.), containing 10% fetal bovine serum (FBS, HiClone, Cytiva Co., Ltd.) and 1% v/v penicillin/streptomycin (Life Technologies Hong Kong Ltd.).

### 1.3 Concentration dependent treatment with the SS-SRGR - (+ 7) compound

The compound was dissolved in DMSO (Sigmaldrich Co Ltd.) at a concentration of 10 mM as a stock. Serum dilutions of the compound in serum-free DMEM were prepared fresh (0, 10, 25 nM) prior to incubation in culture. HT22 cells were seeded on a 6-well plate 24 hours prior to treatment. Cells were incubated in serum-free DMEM for 3 hours prior to treatment with the compound. After incubation, HT22 cells were then incubated with SS-SRGR - (+ 7) for 30 minutes. Cells from different groups were then harvested for protein extraction.

### 1.4 Quantification of proteins in each soluble fraction and western blot analysis.

Each cell sample was homogenized in ice-cold lysis buffer (Cell Signaling Technology, USA), containing a cocktail of protease inhibitors, by ultrasonic homogenizer before being centrifuged at 12,000 × g, 4 °C for 15 min. The supernatant of each sample was collected as a soluble fraction. The protein concentration of each fraction was measured using the BioRad Protein Assay reagent (Bio-Rad Laboratories, Inc.). Detection was performed by western blotting. 20 µg of protein was dissolved in 1X SDS buffer containing 2-mercaptoethanol and heated to 95 °C for 5 minutes. After performing SDS-PAGE (10% SDS gel electrophoresis), the proteins were transferred to a polyvinylidene fluoride (PVDF) membrane (GE Healthcare Corporation). The PVDF membrane was blocked for 45 min with 5% fat-free milk in TBS/0.1% Tween 20 (TBST). The PVDF membrane was then incubated overnight at 4 °C with the primary antibodies diluted in TBST solution. Unbound antibodies were then washed off by incubating the PVDF membrane in TBST with orbital shaking for 15 minutes. Then, the membrane was incubated with secondary antibodies, thus allowing visualization of the antigen-antibody complex using the Westernbright Quantum HRP substrate (Advansta, USA). The densitometric analysis of the bands detected by the chemiluminescent reaction was quantified by the Image J software (NIH: National Institutes of Health). The amounts of target proteins were standardized on the amount of β (beta)-actin proteins.

### 2. Results

FIG. 2A is a western blot analysis image showing the experimental results of pAMPK protein amounts in HT22 cells after induction with the compound. FIGURE 2B is a semi-quantification graph summarizing the experimental results of FIG. 2A. Each error bar represents the standard error of the mean in AMPK phosphorylation, after normalizing to the amount of AMPK protein, which in turn is standardized to (beta) - actin. Statistically, the amounts of pAMPK proteins were observed to increase significantly in a concentration-dependent manner. Since AMPK is an important cytoplasmic mediator of the adiponectin signaling pathway, this experimental result indicated that the compound induced the adiponectin signal in neuronal cells.

### Experiment 2

Suppression of adiponectin receptor expression by siRNA transfection reduced the activation of AMPK by SS-SRGR - (+ 7).

### 1. Materials and methods

### 1.1 Antibodies

A rabbit adiponectin anti-receptor 1 polyclonal antibody (AdipoR1) (Abcam, Cambridge, MA, USA), a rabbit adiponectin anti-receptor 2 polyclonal antibody (AdipoR2) (Boster Biological Technology, USA), an anti-phospho-AMPK (pAMPK) rabbit polyclonal antibody (Cell Signaling Technology, Ltd.), an anti-AMPK rabbit polyclonal antibody (Cell Signaling Technology, Ltd.) and an HRP conjugated mouse monoclonal anti-β (beta)-actin antibody (Cell Signaling Technology, Ltd.) were used as primary antibodies after being suitably diluted. An HRP conjugated goat anti-rabbit IgG antibody (Dako, Glostrup, Denmark) was used as secondary antibody.

### 1.2 Cell culture

Under conditions of 5% CO2 at 37 °C, mouse neuronal cells (HT22) are maintained and expanded in a Dulbecco-modified high-glucose Eagle medium (DMEM, GIBCO, Invitrogen, Life Technologies Hong Kong Ltd.), containing 10% fetal bovine serum (FBS, HiClone, Cytiva Co., Ltd.) and 1% v/v penicillin/streptomycin (Life Technologies Hong Kong Ltd.).

### 1.3 siRNA transfection

SiRNA scramble (non-targeting RNA), siAdipoR1 and siAdipoR2 were purchased from Santa Cruz Biotechnology. siAdipoR1, siAdipoR2 were prepared in DMEM to break down the expression of AdipoR1 and AdipoR2, respectively. SiRNA scramble was prepared as a control for transfection specificity. HT22 neuronal cells were cultured using a commercially available 6-well plate and transfected using Lipofectamine 3000 (Invitrogen, Life Technologies Hong Kong Ltd.) in a 70% confluence state. After transfection for 8 hours, the medium was replaced with a DMEM containing 10% FBS. After 24 hours, the medium was then replaced with serum-free DMEM medium to avoid the effects of adiponectin in serum. After 48 hours, transfected HT22 neuronal cells were harvested to detect adipoR1 or adipoR2 by western blot analysis. Another batch of HT22 neuronal cells transfected with siRNA was treated with the compound (25 nM) for 30 minutes to activate the adiponectin signal. The cells were then harvested for soluble protein extraction after treatment with the compound.

### 1.4 Quantification of proteins in each soluble fraction and western blot analysis.

The extraction of each soluble fraction and the quantification of the proteins in each soluble fraction by western blot analysis were performed according to procedures similar to section 1.4 of Experiment 1.

### 2. Results

Figure 3 is the western blot analysis image showing the protein fractions prepared from HT-22 cells transfected with siAdipoR1 and HT-22 cells transfected with siAdipoR2, respectively. FIG. 3A and 3C indicate that HT22 neuronal cells transfected with siAdipoR1 and siAdipoR2 had a reduced expression of AdipoR1 and AdipoR2, respectively. FIG. 3B and 3D show the semi-quantitative expression of the Adipo-R protein normalized with the amount of protein detected by the anti-actin antibody.

FIG. 4 is the western blot analysis image showing the protein fractions prepared from HT-22 cells transfected with siAdipoR1 and HT-22 cells transfected with siAdipoR2, with or without SS-SRGR - (+ 7), respectively, with the soluble fractions detected using anti-phospho-AMPK antibody (pAMPK). As shown in FIG. 4A and 4C, siAdipoR1 and siAdipoR2 did not reduce the pAMPK level of HT22 cells. However, Adipo-R1 and Adipo-R2 knock-down significantly reduced SS-SRGR (+ 7) -induced AMPK activation, compared to siCtrl-transfected HT22 cells.

Therefore, the results indicated that the promotion of AMPK phosphorylation by SS-SRGR - (+ 7) depended on both AdipoR1 and AdipoR2. SS-SRGR - (+ 7) is a potent mixed agonist (Adipo-R1 and R2) of the adiponectin receptor.

The results of Experiments 1 and 2 teach that the compound of formula (1), i.e. SS-SRGR - (+ 7), should be suitable for the treatment of a disease or disorder capable of being treated by an agonist of AdipoR.

### Experiment 3

A high glucose level is the cause of neuronal apoptosis leading to diabetic neuropathy. To examine whether SS-SRGR - (+ 7) can protect neurons from the hyperglycemic condition, neuronal cells in different glucose concentrations (0, 25, 50, 100 mM) were grown. It was seen that the viability of NSC34 neuronal cells was significantly reduced in the 100 mM glucose (Figure 5A). NSC-34 neurons were then pre-treated with or without SS-SRGR - (+ 7) (25 nM) prior to incubation with 100 mM glucose. Cell viability of SS-SRGR - (+ 7) pretreated NSC34 cells was found to be significantly higher than vehicle-pretreated NSC34 under high glucose conditions. These data suggested that SS-SRGR - (+ 7) protects NSC34 motor neurons from high glucose-induced apoptosis.

The results of Experiments 1, 2, and 3 teach that the compound of formula (1) has dual activity, as a mixed agonist of AdipoR, i.e. AdipoR1 and AdipoR1, and as a neuroprotective agent against high glucose-induced apoptosis of NSC34 motor neurons. This dual activity of the compound of formula (1) means that the compound should be particularly suitable for the treatment of diabetic neuropathy.

## Claims

1. Compound of formula (1) 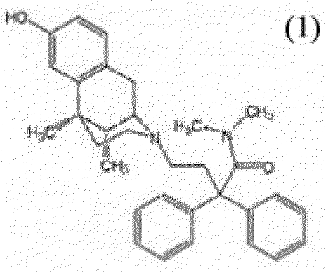 for use in the treatment of a disease or disorder capable of being treated by an agonist of AdipoR wherein the disease or disorder is a metabolic disease or disorder selected from obesity, atherosclerosis, dyslipidaemia, non-alcoholic fatty liver disease, hyperglycaemia and diabetes.

2. Compound of formula (1) for use according to claim 1 wherein AdipoR is AdipoR1 and/or AdipoR2.

3. Compound of formula (1) for use according to any one of claims 1-2 wherein the disease or disorder comprises hyperglycaemia.

4. Compound of formula (1) for use according to any one of claims 1 to 3 wherein the disease or disorder is selected from diabetes, diabetic nephropathy, and diabetic retinopathy

5. Compound of formula (1) for use according to any one of claims 1 to 3 wherein the disease or disorder is diabetic neuropathy.

6. Compound of formula (1) for use according to any one of the preceding claims in mammals.

7. Compound of formula (1) for use according to any one of the preceding claims in humans.

8. Pharmaceutical composition comprising the compound of formula (1) for use according to claim 1 and pharmaceutically acceptable excipients.

9. Pharmaceutical composition for use according to claim 8 in the form of a solution, suspension, powder, granule, tablet, pill, capsule, syrup, suppository, spray, aerosol, controlled-release system, cream, ointment, gel, lubricant, paste, syrup, ampoule, phial, drop or eye drop.

10. Pharmaceutical composition according to claims 8-9 for use in the treatment of a disease or disorder capable of being treated by an agonist of AdipoR wherein the disease or disorder is a metabolic disease or disorder selected from obesity, atherosclerosis, dyslipidaemia, non-alcoholic fatty liver disease, hyperglycaemia and diabetes.

11. Pharmaceutical composition for use according to claim 10 wherein AdipoR is AdipoR1 and/or AdipoR2.

12. Pharmaceutical composition for use according to any one of claims 10-11 wherein the disease or disorder comprises hyperglycaemia.

13. Pharmaceutical composition for use according to any one of claims 10-12 wherein the disease or disorder is selected from diabetes, diabetic nephropathy, and diabetic retinopathy.

14. Pharmaceutical composition for use according to any one of claims 10 to 12 and claim 16 wherein the disease or disorder is diabetic neuropathy

15. Pharmaceutical composition as defined according to claim 8 or claim 9, for use according to any one claims 12 to 14 in mammals.

16. Pharmaceutical composition as defined according to claim 8 or claim 9, for use according to any one of claims 12 to 14 in humans.

17. Pharmaceutical composition as defined according to claim 8 or claim 9 for use according to any one of claims 12 to 16 to be administered orally, transdermally, subcutaneously, intravenously, rectally or intranasally.

## Patentansprüche

1. Verbindung von Formel (1) 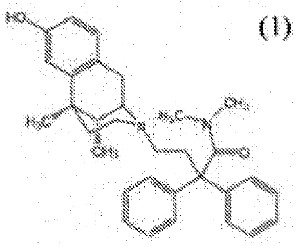 zur Verwendung bei der Behandlung einer Krankheit oder Störung, die mit einem Agonisten von AdipoR behandelt werden kann, wobei die Krankheit oder Störung eine Stoffwechselkrankheit oder -störung ist, die aus Adipositas, Arteriosklerose, Dyslipidämie, nichtalkoholischer Fettlebererkrankung, Hyperglykämie und Diabetes ausgewählt ist.

2. Verbindung von Formel (1) zur Verwendung gemäß Anspruch 1, wobei AdipoR AdipoRl und/oder AdipoR2 ist.

3. Verbindung von Formel (1) zur Verwendung gemäß einem der Ansprüche 1 bis 2, wobei die Krankheit oder Störung Hyperglykämie aufweist.

4. Verbindung von Formel (1) zur Verwendung gemäß einem der Ansprüche 1 bis 3, wobei die Erkrankung oder Störung ausgewählt ist aus Diabetes, diabetischer Nephropathie und diabetischer Retinopathie.

5. Verbindung von Formel (1) zur Verwendung gemäß einem der Ansprüche 1 bis 3, wobei die Erkrankung oder Störung diabetische Neuropathie ist.

6. Verbindung von Formel (1) zur Verwendung gemäß einem der vorangehenden Ansprüche bei Säugetieren.

7. Verbindung von Formel (1) zur Verwendung gemäß einem der vorangehenden Ansprüche bei Menschen.

8. Pharmazeutische Zusammensetzung, die die Verbindung von Formel (1) zur Verwendung gemäß Anspruch 1 und pharmazeutisch verträgliche Hilfsstoffe aufweist.

9. Pharmazeutische Zusammensetzung zur Verwendung gemäß Anspruch 8 in Form einer Lösung, einer Suspension, eines Pulvers, eines Granulats, einer Tablette, einer Pille, einer Kapsel, eines Sirups, eines Suppositoriums, eines Sprays, eines Aerosols, eines Systems mit kontrollierter Freisetzung, einer Creme, einer Salbe, eines Gels, eines Gleitmittels, einer Paste, eines Sirups, einer Ampulle, einer Phiole, eines Tropfens oder eines Augentropfens.

10. Pharmazeutische Zusammensetzung gemäß den Ansprüchen 8-9 zur Verwendung bei der Behandlung einer Krankheit oder Störung, die mit einem Agonisten von AdipoR behandelt werden kann, wobei die Krankheit oder Störung eine Stoffwechselkrankheit oder -störung ist, die aus Adipositas, Arteriosklerose, Dyslipidämie, nichtalkoholischer Fettlebererkrankung, Hyperglykämie und Diabetes ausgewählt ist.

11. Pharmazeutische Zusammensetzung zur Verwendung gemäß Anspruch 10, wobei AdipoR AdipoR1 und/oder AdipoR2 ist.

12. Pharmazeutische Zusammensetzung zur Verwendung gemäß einem der Ansprüche 10-11, wobei die Krankheit oder Störung Hyperglykämie aufweist.

13. Pharmazeutische Zusammensetzung zur Verwendung gemäß einem der Ansprüche 10 bis 12, wobei die Krankheit oder Störung ausgewählt ist aus Diabetes, diabetischer Nephropathie und diabetischer Retinopathie.

14. Pharmazeutische Zusammensetzung zur Verwendung gemäß einem der Ansprüche 10 bis 12 und Anspruch 16, wobei die Krankheit oder Störung eine diabetische Neuropathie ist.

15. Pharmazeutische Zusammensetzung wie gemäß Anspruch 8 oder 9 definiert zur Verwendung gemäß einem der Ansprüche 12 bis 14 bei Säugetieren.

16. Pharmazeutische Zusammensetzung wie gemäß Anspruch 8 oder 9 definiert zur Verwendung gemäß einem der Ansprüche 12 bis 14 bei Menschen.

17. Pharmazeutische Zusammensetzung wie gemäß Anspruch 8 oder 9 definiert zur Verwendung gemäß einem der Ansprüche 12 bis 16 um oral, transdermal, subkutan, intravenös, rektal oder intranasal verabreicht zu werden.

## Revendications

1. Composé de formule (1) : 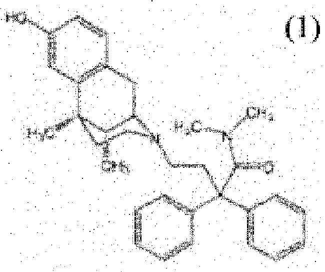 pour une utilisation dans le traitement d'une maladie ou d'un trouble pouvant être traité(e) par un agoniste de l'AdipoR dans lequel la maladie ou le trouble est une maladie ou un trouble métabolique sélectionné(e) parmi l'obésité, l'athérosclérose, la dyslipidémie, la stéatose hépatique non alcoolique, l'hyperglycémie et le diabète.

2. Composé de formule (1) pour une utilisation selon la revendication 1, dans lequel l'AdipoR est l'AdipoR1 et/ou l'AdipoR2.

3. Composé de formule (1) pour une utilisation selon l'une quelconque des revendications 1 à 2 dans lequel la maladie ou le trouble comprend une hyperglycémie.

4. Composé de formule (1) pour une utilisation selon l'une quelconque des revendications 1 à 3 dans lequel la maladie ou le trouble est sélectionné(e) parmi le diabète, la néphropathie diabétique et la rétinopathie diabétique

5. Composé de formule (1) pour une utilisation selon l'une quelconque des revendications 1 à 3 dans lequel la maladie ou le trouble est une neuropathie diabétique.

6. Composé de formule (1) pour une utilisation selon l'une quelconque des revendications précédentes chez les mammifères.

7. Composé de formule (1) pour une utilisation selon l'une quelconque des revendications précédentes chez l'homme.

8. Composition pharmaceutique comprenant le composé de formule (1) pour une utilisation selon la revendication 1 et des excipients pharmaceutiquement acceptables.

9. Composition pharmaceutique pour une utilisation selon la revendication 8 sous forme de solution, suspension, poudre, granulé, comprimé, pilule, gélule, sirop, suppositoire, spray, aérosol, système à libération contrôlée, crème, pommade, gel, lubrifiant, pâte, sirop, ampoule, flacon, goutte ou goutte opthalmique.

10. Composition pharmaceutique selon les revendications 8 à 9 pour une utilisation dans le traitement d'une maladie ou d'un trouble pouvant être traité(e) par un agoniste de l'AdipoR dans laquelle la maladie ou le trouble est une maladie ou un trouble métabolique sélectionné(e) parmi l'obésité, l'athérosclérose, la dyslipidémie, la stéatose hépatique non alcoolique, l'hyperglycémie et le diabète.

11. Composition pharmaceutique pour une utilisation selon la revendication 10 dans laquelle l'AdipoR est l'AdipoR1 et/ou l'AdipoR2.

12. Composition pharmaceutique pour une utilisation selon l'une quelconque des revendications 10 à 11 dans laquelle la maladie ou le trouble comprend une hyperglycémie.

13. Composition pharmaceutique pour une utilisation selon l'une quelconque des revendications 10 à 12 dans laquelle la maladie ou le trouble est sélectionné(e) parmi le diabète, la néphropathie diabétique et la rétinopathie diabétique.

14. Composition pharmaceutique pour une utilisation selon l'une quelconque des revendications 10 à 12 et la revendication 16 dans laquelle la maladie ou le trouble est une neuropathie diabétique.

15. Composition pharmaceutique selon la revendication 8 ou la revendication 9, pour une utilisation selon l'une quelconque des revendications 12 à 14 chez les mammifères.

16. Composition pharmaceutique telle que définie selon la revendication 8 ou la revendication 9, pour une utilisation selon l'une quelconque des revendications 12 à 14 chez l'homme.

17. Composition pharmaceutique telle que définie selon la revendication 8 ou la revendication 9 pour une utilisation selon l'une quelconque des revendications 12 à 16 à administrer par voie orale, transdermique, sous-cutanée, intraveineuse, rectale ou intranasale.
